# EUROPÄISCHE PATENTANMELDUNG

(11) **EP 2 298 372 A1**
(43) Veröffentlichungstag der Anmeldung: **23.03.2011**
(21) Anmeldenummer: 09075440.9
(22) Anmeldetag: 22.09.2009
(51) Int. Cl.: A61M 1/10, A61M 1/12, F04D 29/18, F04D 29/24

(54) **Rotor für eine Axialpumpe zur Förderung eines Fluids**

(71) Anmelder: ECP Entwicklungsgesellschaft mbH, 12247 Berlin (DE)
(72) Erfinder: Schumacher, Jörg, 14513 Teltow (DE); Röhn, Daniel, 12557 Berlin (DE)
(74) Vertreter: Pfenning, Meinig & Partner GbR

(57) **Zusammenfassung**

Die Erfindung bezieht sich auf einen Rotor für eine Axialpumpe zur Förderung eines Fluids mit einer Rotationsachse (10) und mit einem Schaufelblatt (12, 12', 12", 12"'), das wenigstens eine Teilfläche aufweist, die sich quer zur Rotationsachse (10) über diese hinweg erstreckt, wobei das Schaufelblatt durchgehende Stege (15,15', 15" , 15"', 16, 16', 16", 16"', 17) bzw. ein Netzwerk von Stegen aufweist, das verschiedene Randbereiche des Schaufelblattes miteinander verbindet. Hierdurch wird eine gute Komprimierbarkeit in radialer Richtung mit hoher Stabilität während des Betriebs verbunden.

## Beschreibung

Die Erfindung liegt auf dem Gebiet des Maschinenbaus, insbesondere der Feinwerktechnik, und ist mit besonderem Gewinn auf dem medizinischen Gebiet anwendbar.

Spezieller bezieht sich die Erfindung auf einen Rotor für eine Axialpumpe.

Insbesondere in der Medizintechnik werden für vielfältige Anwendungen Pumpen in kleinen Bauformen, sogenannte Mikropumpen, benötigt. Diese werden für mikroinvasive Anwendungen, beispielsweise zur Förderung körpereigener Flüssigkeiten in körpereigenen Hohlräumen bzw. Gefäßen eingesetzt. Üblicherweise werden solche Pumpen in Mikrobauform mit Kathetern verbunden und beispielsweise durch körpereigene Gefäße eingeführt und an den Anwendungsort gebracht. Ein spezielles Beispiel zum Einsatz derartiger Pumpen sind die sogenannten Herzpumpen, die durch ein großes Blutgefäß in den Körper eingeführt werden und die Blutförderung des Herzens unterstützen oder sogar ersetzen können.

Speziell sind in diesem Zusammenhang Rotationspumpen bekannt geworden, die als Axialpumpen ausgeführt sind.

Eine spezielle Eigenschaft mancher derartiger Pumpen ist außer ihrer an sich kleinen Bauform weiter die radiale Komprimierbarkeit, so dass eine derartige Pumpe zum Transport durch ein Blutgefäß komprimiert und nach dem Verbringen an den Einsatzort beispielsweise in einer Herzkammer expandiert werden kann.

Eine derartige Pumpe ist beispielsweise aus den US-Offenlegungsschriften US 2009/0060743 A1 und US 2008/0114339 A1 bekannt geworden. Die in diesen Dokumenten beschriebenen Axialpumpen weisen jeweils eine Welle und daran flexible befestigte Schaufelblattreihen auf, die bei Rotation der Welle in axialer Richtung ein Fluid fördern. Die Schaufelblätter sind radial an die Welle anlegbar, so dass auf diese Weise der Rotor komprimierbar ist. Im Betrieb richten sich die einzelnen Schaufelblätter unter anderem durch den Fluidgegendruck auf, so dass die Pumpe eine beträchtliche Förderkapazität aufweist.

Der vorliegenden Erfindung liegt vor dem Hintergrund des Standes der Technik die Aufgabe zugrunde, einen Rotor für eine Axialpumpe zu schaffen, der möglichst einfach aufgebaut und kostengünstig herstellbar ist. Er soll außerdem massearm sein und eine ausreichende Förderleistung ermöglichen.

Die Aufgabe wird mit dem Merkmalen der Erfindung gemäß Patentanspruch 1 gelöst.

Der erfindungsgemäße Rotor weist ein Schaufelblatt mit wenigstens einer Teilfläche auf, die sich quer zur Rotationsachse über diese hinweg erstreckt, wobei das Schaufelblatt Stege aufweist, die jeweils einzeln oder als Netzwerk verschiedene Randbereiche des Schaufelblattes miteinander verbinden.

Das kann zum Beispiel bedeuten, dass wenigstens eine Teilfläche des Schaufelblattes derart gestaltet ist, dass Elemente der Teilfläche einander bezüglich der Rotationsachse auf derselben axialen Position auf verschiedenen Seiten der Rotationsachse gegenüberliegen. Dies kann beispielsweise derart realisiert sein, dass die Rotationsachse die Teilfläche durchsetzt und radial zu mehreren Seiten von Elementen der Teilfläche umgeben ist. Dieses Beispiel kann auch derart beschrieben werden, dass der Teilfläche ein Kreis einbeschrieben werden kann, den die Rotationsachse mittig durchsetzt.

Dabei weist das Schaufelblatt durchgehende Stege oder ein Netzwerk von Stegen auf, die verschiedene Randbereiche des Schaufelblattes, die auch als Randabschnitte bezeichnet werden können, miteinander verbinden und somit das Schaufelblatt aufspannen. Derartige Stege sind dazu geeignet, insbesondere gemeinsam mit gegebenenfalls vorgesehenen Randleisten des Schaufelblattes dieses aufzuspannen und die Befestigung einer Folie zu erlauben, die die Förderfläche des Schaufelblattes bildet und von den Stegen gestützt ist. Damit wird eine sehr leichte Bauart des Schaufelblattes realisiert, die dennoch die Schaffung einer großen Förderfläche erlaubt.

Das Schaufelblatt ist vorteilhaft nabenlos gestaltet und selbsttragend realisiert.

Das bedeutet, dass das Drehmoment entlang des Rotors mittels der Schaufelblattfläche, also des flachen, gebogenen Körpers übertragen wird, der das Schaufelblatt im Wesentlichen bildet. Da die üblicherweise zur Übertragung des Drehmomentes und zur Halterung der Schaufelblattfläche vorgesehene Nabe ein wesentliches Volumen aufweist, das mit der Konstruktion gemäß der Erfindung eingespart werden kann, ist eine wesentlich stärkere Komprimierbarkeit des Rotors gemäß der Erfindung erreichbar.

Wenigstens einer der Stege kann zwei Randbereiche des Schaufelblattes verbinden, die einander radial bezüglich der Rotationsachse gegenüberliegen.

Es kann jedoch auch vorgesehen sein, dass wenigstens ein Steg zwei Randbereiche des Schaufelblattes verbindet, die in Längsrichtung der Rotationsachse gesehen einander gegenüberliegen.

In jedem Fall kann die Gesamtfläche des Schaufelblattes nach einem gewünschten Muster durch Stege unterteilt sein, um eine gewünschte Schaufelblattfläche zu schaffen, die entweder durch die Stege selbst gebildet wird oder durch eine über die Stege gespannte Folie. Die Stege müssen ebenso wie die Förderfläche des Schaufelblattes nicht in einer Ebene verlaufen, sondern können eine dreidimensional, bespielsweise als Schraubenwendel verlaufende Fläche beschreiben. Dabei können die Stege auch punktweise an Knotenpunkten des Schaufelblattes miteinander verbunden sein, beispielsweise an solchen Punkten, die einer besonderen mechanischen Belastung ausgesetzt sind. Derartige Knotenpunkte können allerdings auch derart gewählt werden, dass durch sie ein Falten der Stege bei einer Kompressions- oder Expansionsbewegung des Schaufelblattes ermöglicht oder erleichtert wird.

Um die Stege entsprechend komprimierbar bzw. expandierbar zu gestalten, können sie vorteilhaft mäanderförmig gestaltet werden. Vorteilhaft ist die Mäanderstruktur in der Fläche des Schaufelblattes angelegt.

Um einen entsprechenden Mechanismus zur Komprimierung und Expandierung des Schaufelblattes zu schaffen, kann vorteilhaft vorgesehen sein, dass die Stege aus einer Formgedächtnislegierung, beispielsweise Nitinol, bestehen. In diesem Fall kann durch Temperaturwechsel jeweils eine gewünschte Gestalt des Schaufelblattes angestrebt werden. Auch kann der Mechanismus des Komprimierens hier durch Ausnutzung de hyperelastischen Eigenschaften des Werkstoffes Nitinol unterstützt werden.

Wenn Randabschnitte des Schaufelblattes als Randleisten ausgebildet sind, so können diese das Schaufelblatt zusätzlich stabilisieren und gemeinsam mit den Stegen einen zuverlässigen Halt bzw. eine Stütze für eine entsprechende Schaufelblattfolie bilden. Eine derartige Folie kann dann an den Stegen und an den Randleisten oder Teilen der Randleisten beispielsweise durch Kleben befestigt sein.

Die Erfindung bezieht sich auch auf einen Rotor für eine Axialpumpe zur Förderung eines Fluids mit einer Rotationsachse und mit einem Schaufelblatt, welches nabenlos als ein bezüglich seiner Kontur flacher Körper gestaltet ist, der um eine Achse spiralförmig verdreht ist.
Diese Konstruktionsart lässt eine besonders einfache Herstellung zu und ist, insbesondere dadurch, dass keine Nabe benötigt wird, besonders einfach und auf ein besonders kleines Maß komprimierbar. Dies ist zum Einführen des Rotors beispielsweise über eine Blutbahn im Körper eines Menschen für medizinische Anwendungen entscheidend.

Vorteilhaft ist das Schaufelblatt als ein Gitterwerk oder Netzwerk von Stegen aus einem ebenen Blech hergestellt.

Dies lässt eine Kostengünstige Serienherstellung mit gängigen Methoden der Blechbearbeitung zu.

Speziell kann vorteilhaft vorgesehen sein, dass das Schaufelblatt insbesondere aus einem Nitinolblech, durch Ausschneiden der Stege insbesondere durch Wasserschneiden, Laserschneiden oder Elektroerodieren hergestellt ist.

Die Stege können dabei in der Blechebene und/oder senkrecht dazu mäanderförmig geformt sein.
Dadurch ergibt sich eine leichte Biegbarkeit bei der Kompression des Rotors in Radialrichtung.

Weiterhin können die Stege in der Blechebene ein anderes Flächenträgheitsmoment aufweisen als senkrecht dazu.

Dadurch kann gegenüber einer radialen Kompression des Schaufelblattes ein wesentlich geringerer widerstand realisiert werden als gegenüber Belastungen, die durch den Pumpbetrieb auf das Schaufelblatt wirken. Belastungen, die durch einen Fluiddruck gegen die Schaufelblattebene entstehen, werden somit sehr viel steifer aufgenommen.

Eine besonders einfache Ausgestaltung eines erfindungsgemäßen Schaufelblatts sieht vor, dass dieses als ein länglicher, insbesondere rechteckiger Körper gestaltet ist, der um eine Achse, insbesondere seine Längsmittelachse spiralförmig verdreht ist. Dabei kann die Spiralform auch unregelmäßig bezüglich der Steigung ausgestaltet oder gegebenenfalls anderweitig verzerrt sein.

Die Verdrehungsachse des Körpers liegt beim Zusammenbau des Rotors vorzugsweise im Wesentlichen parallel zur Rotationsachse oder fällt mit dieser zusammen.

Damit ergibt sich eine symmetrische oder begrenzt unsymmetrische wendelförmige Gestaltung eines Schaufelblattes, beispielsweise dadurch, dass die Enden eines ebenen Rechtecks um 180 Grad oder um einen anderen Winkelbetrag um die Längsachse gegeneinander verdreht sind. Die Schaufelblattfläche ist dann als eine einzige, zusammenhängende Fläche ausgebildet, die sich über die Rotationsachse hinweg erstreckt und von dieser durchsetzt ist. Die Fläche kann dabei auch Ausnehmungen, beispielsweise im Bereich der Rotationsachse aufweisen.

Ein derartiges Schaufelblatt kann bei entsprechend stabiler Gestaltung der Stege und Ränder selbsttragend ausgebildet sein, so dass beispielsweise das Drehmoment über das Schaufelblatt allein übertragen werden kann und keine Nabe benötigt wird. Die Steifheit des Schaufelblattes selbst reicht für die Förderung des Fluids aus, wenn dies von einem seiner Enden her angetrieben wird. Das Drehmoment wird dann über den stirnseitigen Rand des Schaufelblattes eingeleitet.

Es kann jedoch vorteilhaft auch vorgesehen sein, dass das Schaufelblatt mit einem dieses umgebenden hohlzylindrischen Bauteil fest verbunden ist. Ein derartiges hohlzylindrisches Bauteil kann beispielsweise als Ring oder Schlauchabschnitt vorgesehen sein, der das Schaufelblatt zusätzlich stabilisiert und mit diesem einstückig hergestellt sein kann. Es können jedoch auch mehrere koaxiale und axial zueinander beabstandete Ringe am Umfang des Rotors mit dem Schaufelblatt verbunden sein.

Diese Ringe können dann axial zueinander durch Stege beabstandet und in sich radial komprimierbar ausgestaltet sein, um zum Zwecke der Einbringung in einen Körper zusammen mit dem Schaufelblatt kollabiert werden zu können.

Die vorliegende Erfindung gestattet die einfachste Herstellung eines Schaufelblatts für eine Axialpumpe, bei der die Ränder und Verstärkungsstege des Schaufelblattes einstückig beispielsweise durch Spritzguss oder Bearbeitung eines Bleches hergestellt und mit einer Folie versehen sein können. Auch entsprechend sich axial an das Schaufelblatt anschließende Abschnitte können einstückig mit dem Schaufelblatt hergestellt sein, um axial an das Schaufelblatt anschließend eine drehbare Lagerung und die Einleitung eines Drehmoments zu ermöglichen.

Im Folgenden wird die Erfindung anhand eines Ausführungsbeispiels in einer Zeichnung gezeigt und anschließend erläutert. Dabei zeigt
- Fig. 1: im Schnitt eine Übersicht über einen in eine Herzkammer eingeführten Herzkatheter mit einer Axialpumpe,
- Fig. 2: ein Schaufelblatt einer Axialpumpe in drei- dimensionaler Ansicht,
- Fig. 3: das Schaufelblatt aus Fig. 2, wobei un- sichtbare Konturen eingezeichnet sind,
- Fig. 4: das Schaufelblatt aus Fig. 2 mit einer Her- vorhebung der sichtbaren Oberfläche durch Schraffur,
- Fig. 5: eine Seitenansicht des Schaufelblattes aus Fig. 2,
- Fig. 6: einen Schnitt der Ansicht aus Fig. 5,
- Fig. 7: eine Ausgestaltung eines Rotors einer Axialpumpe in dreidimensionaler Ansicht,
- Fig. 8: die Ansicht aus Fig. 7 mit eingezeichneten unsichtbaren Konturen sowie
- Fig. 9: eine teilweise durchbrochene Sicht der An- ordnung aus Fig. 7,
- Fig. 10: einen weiteren Rotor, bei dem die Befesti- gung einer Antriebswelle anders gelöst ist als bei der Ausführung gemäß Fig. 7,
- Fig. 11: einen Rotor mit zwei beiderseits befestig- ten Wellenansätzen,
- Fig. 12: eine Seitenansicht eines Rotors mit mäan- derförmigen bzw. gewellten Stegen, die das Schaufelblatt aufspannen,
- Fig. 13: das Schaufelblatt aus Fig. 12 in einer um 90 Grad gedrehten Ansicht,
- Fig. 14: das Schaufelblatt aus Fig. 12 in einer dreidimensionalen Ansicht,
- Fig. 15: das Schaufelblatt aus Fig. 12 in einer axi- alen Draufsicht,
- Fig. 16: eine andere Variante eines Schaufelblattes mit im Wesentlichen in Richtung der Rota- tionsachse verlaufenden Stegen in einer Seitenansicht,
- Fig. 17: die Anordnung aus Fig. 16 in einer um 90 Grad gedrehten Seitenansicht,
- Fig. 18: die Anordnung aus Fig. 16 in einer drei- dimensionalen Ansicht,
- Fig. 19: die Anordnung aus Fig. 16 in einer axialen Draufsicht,
- Fig. 20: eine weitere Ausführungsform eines Rotors mit quer zur Rotationsachse gerade ver- laufenden Stegen in einer Seitenansicht,
- Fig. 21: die Anordnung aus Fig. 20 in einer um 90 Grad gedrehten Seitenansicht,
- Fig. 22: die Anordnung aus Fig. 20 in einer drei- dimensionalen Ansicht,
- Fig. 23: die Anordnung aus Fig. 20 in einer axialen Draufsicht,
- Fig. 24: eine weitere Ausführungsform eines Rotors mit quer zur Rotationsachse verlaufenden, gebogenen Stegen in einer Seitenansicht,
- Fig. 25: die Ausführungsform gemäß Fig. 24 in einer um 90 Grad gedrehten Seitenansicht,
- Fig. 26: die Ausführungsform gemäß Fig. 24 in einer dreidimensionalen Ansicht und
- Fig. 27: eine Draufsicht auf die Anordnung gemäß Fig. 24 in axialer Richtung.

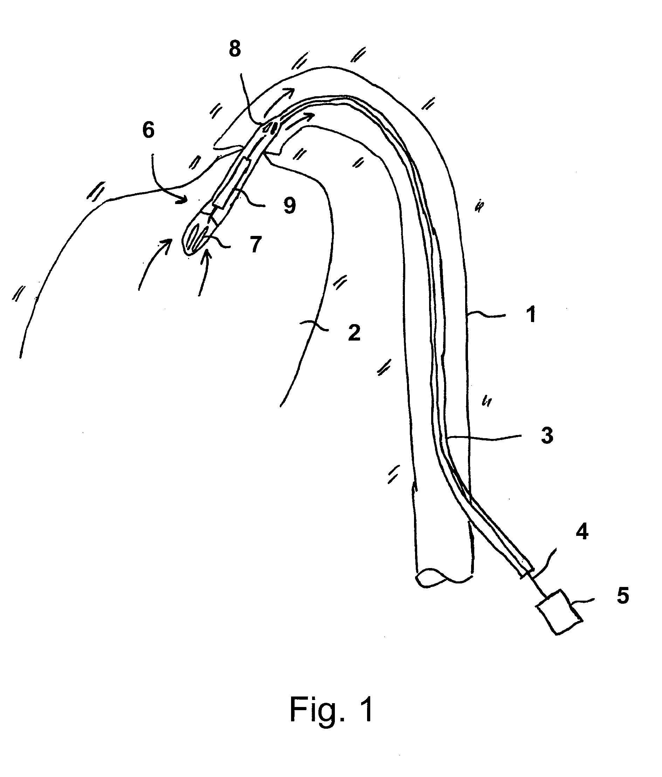

Fig. 1 zeigt schematisch ein Blutgefäß 1 in einem menschlichen Körper, das in einer Herzkammer 2 endet und in das ein Hohlkatheter 3 eingeführt ist.

Durch den Hohlkatheter 3 verläuft eine antreibbare Welle 4, die durch einen außerhalb des Körpers angeordneten Motor 5 mit hoher Drehzahl angetrieben werden kann. Der Hohlkatheter 3 kann mit einer körperverträglichen Flüssigkeit gefüllt sein, die einerseits der Verminderung der Reibung der Welle und andererseits der Abführung von Wärme dienen kann.

Am Ende des Hohlkatheters 3 ist eine Herzpumpe 6 angeordnet, die durch erste Öffnungen 7 innerhalb der Herzkammer 2 Blut ansaugt und dieses über zweite Öffnungen 8 innerhalb des Blutgefäßes 1 wieder abgibt. Auf diese Weise unterstützt die Pumpe 6 die Pumptätigkeit des Herzens oder ersetzt diese.

Im Inneren der Pumpe 6 ist schematisch ein Rotor 9 dargestellt, der um seine Längsachse, angetrieben durch die Welle 4, rotiert und in axialer Richtung von der Herzkammer 2 zum Blutgefäß 1 hin das Blut fördert. Typischerweise ist eine derartige Axialpumpe mit einem Gehäuse und einem darin gelagerten Rotor mit Förderschaufeln versehen.

Derartige Herzpumpen sind bereits in verschiedenen Bauformen bekannt, wobei insbesondere die radiale Komprimierbarkeit solcher Pumpen für ihre Leistungsfähigkeit eine große Rolle spielt. Die Pumpen sollen in komprimierter Form durch das Blutgefäß 1 einbringbar und daraufhin expandierbar sein, damit die Förderschaufeln mit möglichst großen Förderflächen und in einem ausreichend großen Strömungsquerschnitt das Blut fördern können. Hierzu sind verschiedene Rotorkonstruktionen mit faltbaren Rotoren und Gehäusen bereits bekannt. Der erfindungsgemäße Rotor wird anhand der folgenden Figuren genauer geschildert.

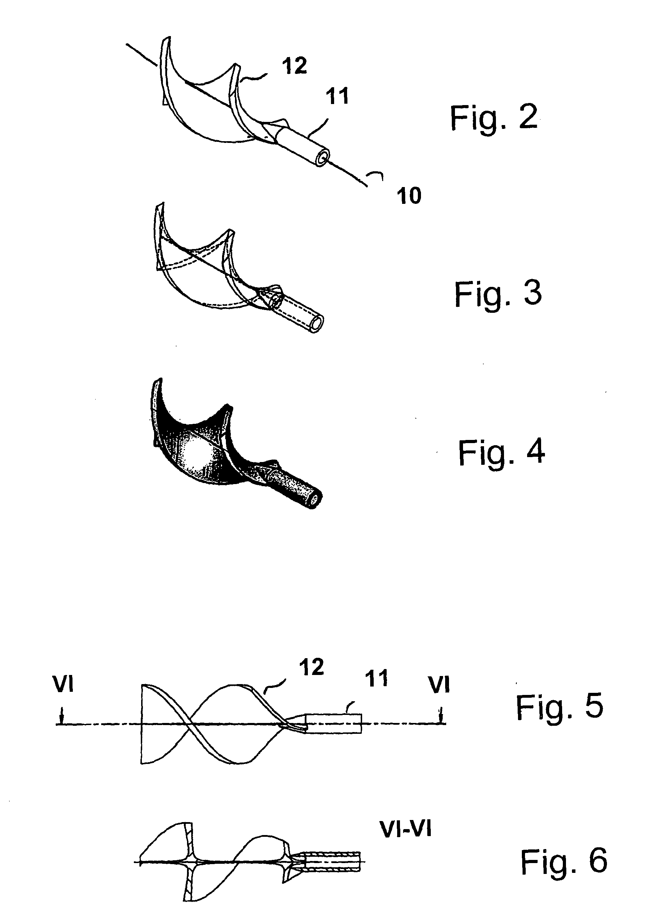

Fig. 2 zeigt dazu zunächst eine Ausführungsform eines einstückigen Schaufelblattes, das wendelförmig um eine Rotationsachse 10 gedreht ist. Es ist eine Welle 11 vorgesehen, die axial an das Schaufelblatt 12 anschließt, dieses jedoch nicht durchsetzt. Das Schaufelblatt 12 ist insoweit selbsttragend und überträgt das Drehmoment, ohne dass eine Nabe notwendig ist.

Das Schaufelblatt 12 kann einstückig mit dem Wellenansatz 11 und gegebenenfalls mit einem weiteren Wellenansatz auf der axial gegenüberliegenden Seite des Schaufelblattes 12 hergestellt sein, beispielsweise in einem Spritzgussverfahren aus Kunststoff.

Die Fig. 2 zeigt schematisch die äußere Form des Schaufelblattes 12, ohne auf den inneren Aufbau näher einzugehen. Dieser wird im Rahmen der Erfindung anhand weiter unten folgender Figuren genauer beschrieben.

Fig. 3 zeigt das Schaufelblatt aus Fig. 2 aus derselben Perspektive, wobei jedoch an sich unsichtbare Linien gestrichelt dargestellt sind. Fig. 4 zeigt eine Darstellung, bei der anhand von Schraffuren die dreidimensionale Form plastischer dargestellt ist.

Fig. 5 zeigt eine Seitenansicht des Schaufelblattes 12 und des Wellenansatzes 11, wobei ein Schnitt mit VI angedeutet ist, der genauer in der Fig. 6 dargestellt ist.

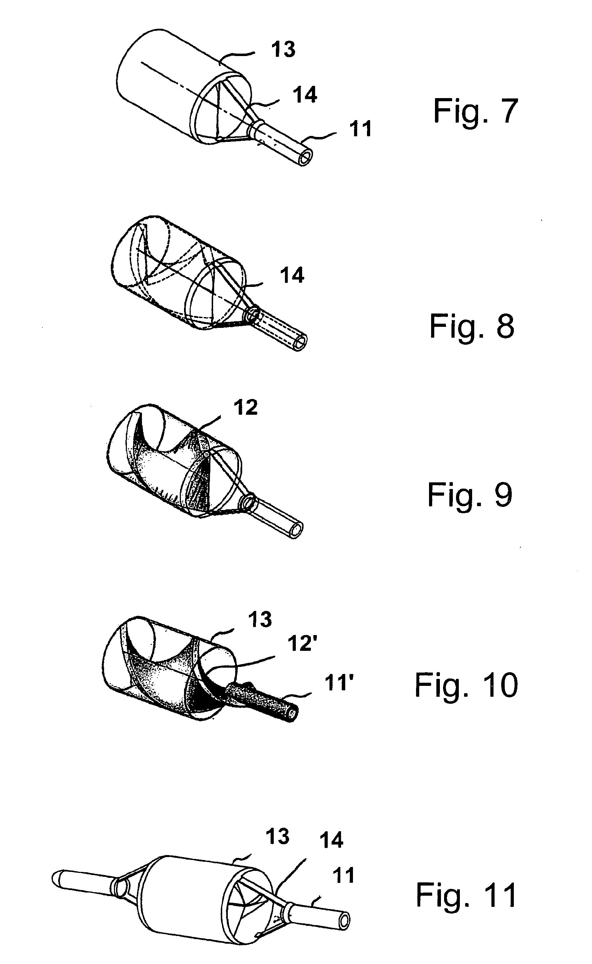

Fig. 7 zeigt eine andere Ausführungsform eines Rotors, bei dem das Schaufelblatt 12 von einer schlauchartigen Stützeinrichtung oder Hülle umgeben ist, mit der es in dieser Ausführungsform starr verbunden ist, so dass die schlauchförmige Hülle oder Stützeinrichtung 13 mit dem Schaufelblatt 12 rotiert.
Die Hülle ist mittels einer gabelförmigen Halterung 14 mit dem Wellenansatz 11 verbunden. Die Halterung kann auch als räumlich verdrehte dreieckige Platte ausgeführt sein, die mit dem Ende des Schaufelblattes 12 direkt verbunden sein kann. Die Hülle ist vorteilhaft komprimierbar und expandierbar und verleiht dem Schaufelblatt 12 Halt. Beispielsweise kann die Hülle 13 aus einem Kunststoffschlauchstück bestehen, das von einem

Maschendrahtgeflecht zur Stützung umgeben sein kann.

Das Maschendrahtgeflecht kann auch aus einem Formgedächtnismaterial bestehen, so dass dieses über seine Formveränderungen die Hülse 13 formgebend stützen kann. Insbesondere in dem Fall, dass das Schaufelblatt 12 nabenlos ausgebildet und nicht selbsttragend stabilisiert ist, kann dieses mit den Innenseiten der Hülse 13 verbunden sein und durch deren Expansionsbewegung aufgespannt werden.

Fig. 8 zeigt die Ansicht aus Fig. 7, wobei an sich unsichtbare Linien gestrichelt eingezeichnet sind, und Fig. 9 zeigt eine dreidimensionale Darstellung des Schaufelblattes 12, wobei die Form durch Schraffuren hervorgehoben ist.

Fig. 10 zeigt als weitere Variante ein von einer Hülse 13 umgebenes Schaufelblatt 12', das einen in seine Form integrierten Wellenansatz 11' aufweist, der für sich nicht mit der Hülse 13 verbunden ist.

Fig. 11 zeigt eine Ausführungsform einer Hülse 13 mit zwei Wellenansätzen 11 zu beiden Seiten, die jeweils über eine gabelförmige Halterung 14 mit der Hülse 13, nicht jedoch mit dem Schaufelblatt, verbunden sind.

Wie in den Figuren 2 bis 10, so ist auch hier ersichtlich, dass das Schaufelblatt einen im Wesentlichen gleichbleibenden Querschnitt ohne Verdickungen aufweisen kann; Querschnittsänderungen sind allerdings keinesfalls ausgeschlossen. Entsprechend wird das Drehmoment über den flächigen Körper selbst übertragen, weshalb keine Nabe benötigt wird.

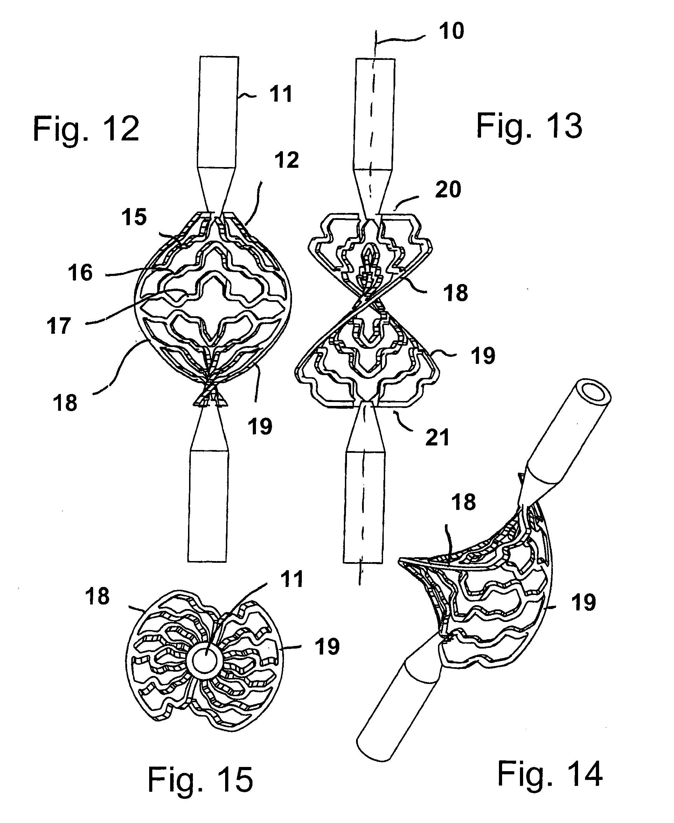

Fig. 12 zeigt genauer den Aufbau eines typischen Schaufelblattes 12, das durch Stege 15, 16, 17 aufgespannt ist. Zudem sind Randleisten 18, 19 eingezeichnet, die typischerweise aus demselben Material bestehen können wie die Stege 15, 16, 17. Die einzelnen Stege sind gewellt ausgeführt, wobei die jeweilige Wellenkontur jeweils innerhalb der Schaufelblattfläche bleibt. Dadurch sind die Stege in der Fläche des Schaufelblatts spannbar und damit expandierbar und komprimierbar. Außerdem ergibt diese Wellenstruktur eine Versteifung senkrecht zur Schaufelblattfläche.

Die Stege können beispielsweise aus einem Formgedächtnismaterial wie Nitinol bestehen, was die Komprimierung und Expandierung des Schaufelblattes 12 zusätzlich erleichtert.

Grundsätzlich besteht das Schaufelblatt 12 in dem dargestellten Beispiel aus einem im Wesentlichen rechteckigen Rahmen, dessen endseitige Randleisten 20, 21, eingezeichnet in Fig. 13, um 180 Grad um die Rotationsachse 10 gegeneinander verdreht sind, um eine wendelförmige Struktur auszubilden. Hierdurch ergibt sich eine einzige, zusammenhängende Fläche, die sich radial zu allen Seiten der Rotationsachse 10 erstreckt und auf jeder Höhe der Rotationsachse Schaufelblattbereiche aufweist, die einander bezüglich der Rotationsachse 10 gegenüberliegen. Hierdurch wird eine hohe Symmetrie des Schaufelblattes mit entsprechend symmetrischer Kraftverteilung erreicht. Grundsätzlich kann der Ausgangskörper auch andere Grundformen als die rechteckige aufweisen, wobei vorteilhaft ist, wenn der Körper später in gewendelter Form den Querschnitt eines Rotorgehäuses möglichst weitgehend abdeckt und mit seiner Außenkontur möglichst genau die Innenkontur des Gehäuses nachbildet. In der Ausführungsform der Fign. 12, 13, 14, 15 können die Wellenansätze 11 einstückig oder durch eine Schweißverbindung mit den Stegen 15, 16, 17 und den Randleisten 18, 19, 20, 21 zusammenhängen, so dass der gesamte Rotor besonders einfach und kostengünstig hergestellt werden kann und verlässliche Verbindungen zur Übertragung des Drehmomentes bestehen. Das aus den Stegen 15, 16, 17 und den Randleisten 18, 19, 20, 21 gebildete Gerüst ist typischerweise mit einer dünnen, hochflexiblen Folie bespannt, die die eigentliche Förderfläche bildet.

Durch das Fehlen einer Nabe in dem axialen Bereich des Schaufelblattes 12 wird einerseits die Komprimierung erleichtert, da die Stege sich auf ihrer gesamten Länge verformen können, andererseits wird der Rotor biegsam, wodurch das Einführen in komprimierter Form längs eines Blutgefäßes erleichtert werden kann. Gemäß der beschriebenen Ausführungsform sind die Stege stellenweise in Knotenpunkten verbunden und bilden ein Netzwerk, das dem Schaufelblatt zusätzliche Steifigkeit verleiht.

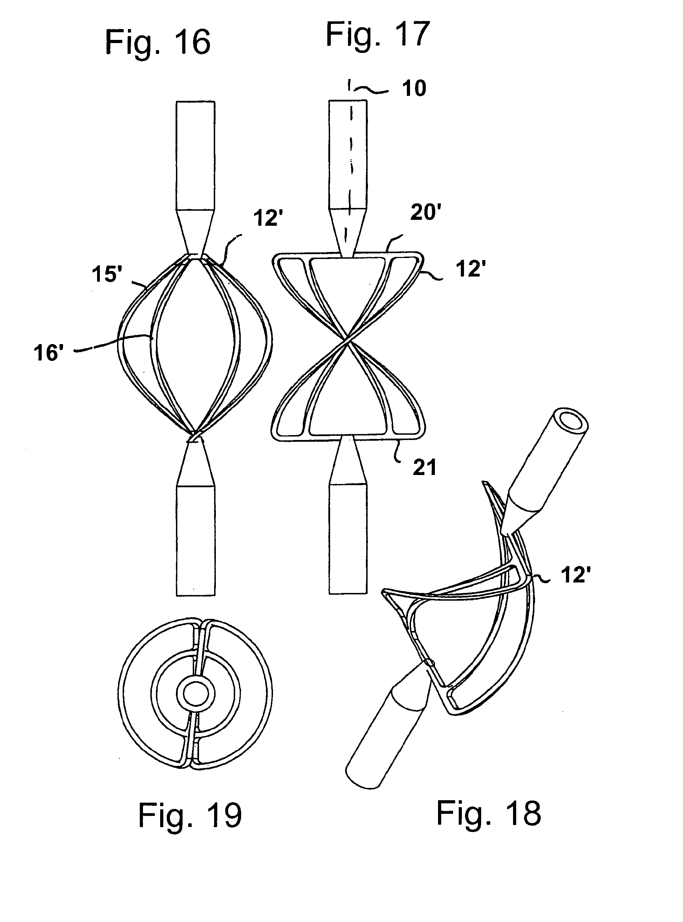

Die Fig. 16, 17 und 18 zeigen in zwei Seitenansichten sowie in einer dreidimensionalen Ansicht einen Rotor mit einem Schaufelblatt 12', bei dem die einzelnen Stege 15', 16' im Wesentlichen entlang der Rotationsachse 10 und dabei wendelförmig um diese herum verlaufen. Es ergibt sich hierdurch eine gute Komprimierbarkeit in radialer Richtung für den Rotor und mit der entsprechenden Bespannung eine Förderfläche, die für das zu fördernde Fluid nur wenige Unregelmäßigkeiten aufweist, so dass auch bei hohen Drehzahlen nur eine relativ geringe Schädigung von Blutbestandteilen zu befürchten ist. Auch bei hohem Fluidgegendruck, der sich bei hohen Rotordrehzahlen einstellen kann, sind Ausweichbewegungen des Schaufelblattes 12' eng begrenzt durch eine gute Stabilisierung der Stege. Auch bei dieser Ausführungsform kann das Schaufelblatt im Ganzen als ein rechteckiger Rahmen angesehen werden, dessen zwei gegenüberliegende stirnseitige Randleisten 20', 21' um 180 Grad um die Rotationsachse 10 gegeneinander verdreht sind. Die Herstellung eines entsprechenden ebenen Rahmens mit parallelen Stegen 15', 16' ist besonders einfach.

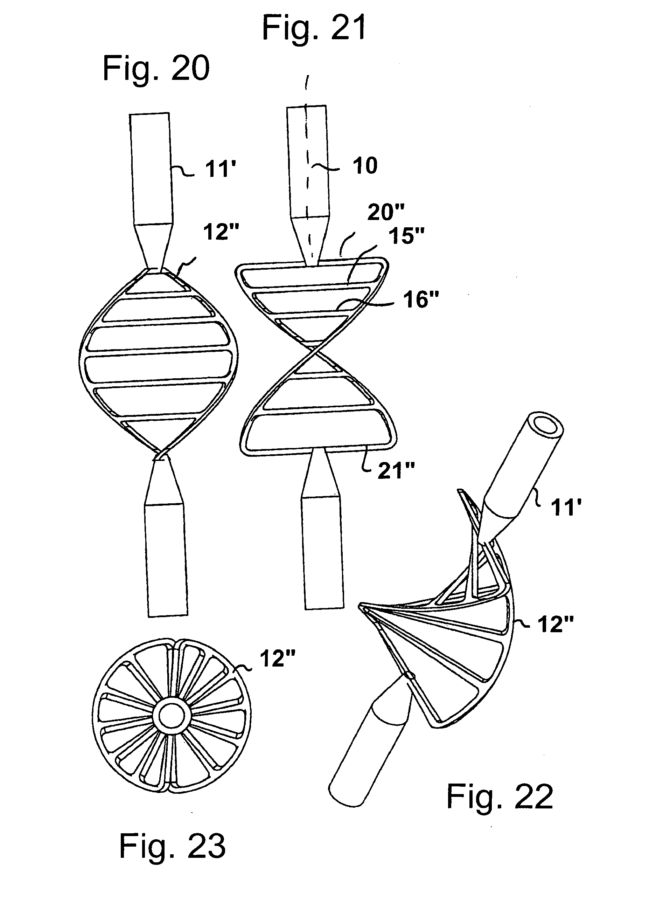

Die Fign. 20, 21, 22 zeigen in zwei Seitenansichten und in dreidimensionaler Ansicht ein Schaufelblatt 12" mit zwei Wellenansätzen 11', wobei das Schaufelblatt horizontal quer zur Rotationsachse 10 verlaufende Stege 15", 16" aufweist, die in sich jeweils gerade sind, dem Schaufelblatt 12" als Ganzem jedoch dieselbe wendelförmige Struktur verleihen, wie sie bezüglich der Kontur bei dem Schaufelblatt gemäß Fig. 17 gegeben ist. Insbesondere wenn das Schaufelblatt 12" durch eine äußere Hülle gestützt und mit dieser verbunden ist, können die Stege 15", 16" des Schaufelblattes 12" bei der Expansionsbewegung mit dem Ergebnis eines stabilen Schaufelblattes besonders effizient gestreckt werden. Dadurch wird die zwischen den Stegen und den Randleisten 20", 21" gespannte Folie ebenfalls stabilisiert, so dass sie faltenfrei in dem zu fördernden Fluid steht.

Fig. 23 zeigt dazu eine stirnseitige Ansicht des Rotors mit den über die Rotationsachse hinaus verlaufenden Stegen.

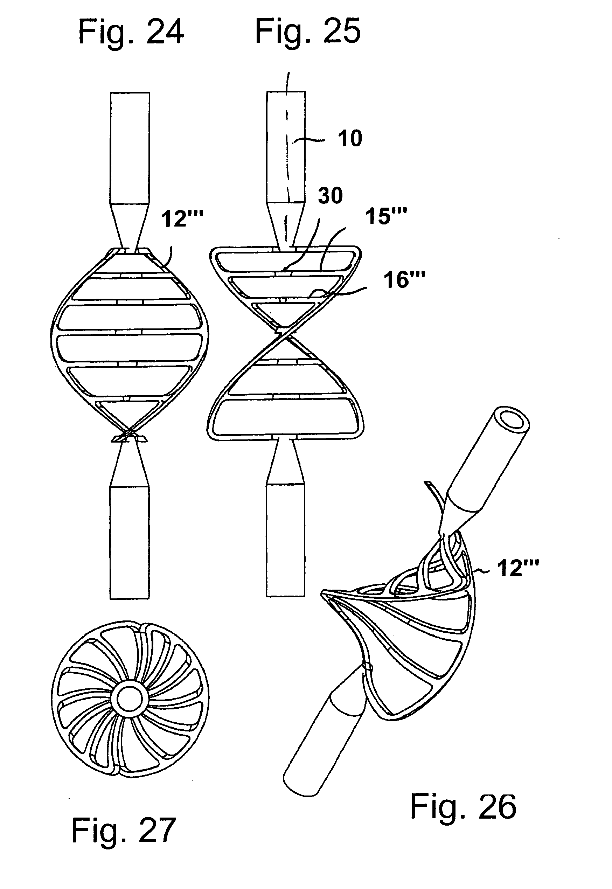

Die Fign. 24-27 zeigen eine Ausführungsform ähnlich der in den Fign. 20-23 gezeigten, mit einem Schaufelblatt 12"', dessen Stege 15"', 16"' quer zur Rotationsachse 10 verlaufen, wobei die einzelnen Stege 15"', 16"' in sich nicht gerade verlaufen, sondern zur Erzielung einer gegebenenfalls weiter verbesserten Schaufelblattgeometrie wellenförmig gebogen sind. Dies kann beispielsweise durch Einfügen eines geschwächten Bereichs 30 jeweils in der Mitte jedes Steges 15"', 16"' erreicht werden, der ein Ausweichen jedes
Steges aus der geraden Richtung bei Einnehmen der dargestellten Wendelform des Schaufelblattes 12"' erleichtert. Es kann aber auch durch eine definierte Vorbiegung der Stege erreicht werden. Der hierdurch erzielte Vorteil ist einerseits, dass die Stege beim Komprimieren eine definierte Vorzugsrichtung einnehmen, so dass es beim Komprimieren nicht zu undefinierten Knickbelastungen der Stege kommt. Ein weiterer Vorteil besteht darin, dass sich die vorgekrümmten Stege wenn sie im Betriebszustand dem Fluiddruck ausweichen, was bei derart elastischen Strukturen nahezu unvermeidbar ist, eine zunehmend gerade Form annehmen. Der Rotor der Fig. 24 bis 27 könnte dann beispielsweise im Betriebszustand die Form des Rotors der Fig. 20 bis 23 einnehmen.
Auch bei dieser Ausführungsform, wie bei den übrigen oben geschilderten, ist das Schaufelblatt insgesamt von Randleisten zum Befestigen der die Förderfläche bildenden Folie und zur Stabilisierung des Schaufelblattes umgeben.

Grundsätzlich erlaubt das in den Fig. 12 bis 26 dargestellte Konstruktionsprinzip eine beliebige Ausbildung der Stege, so dass hier umfangreiche Optimierungsmöglichkeiten bestehen, um das Muster den Anforderungen entsprechend auszugestalten.

In einer vorteilhaften Ausführungsform sind die dabei die Stege so ausgebildet, dass bei der Verformung des Rotors in die vorgesehene komprimierte Form ausschließlich elastische Verformungen auftreten, so dass sich der Rotor nach Wegfall der die Komprimierung auslösenden Kräfte selbsttätig in die vorgesehene dekomprimierte Form entfalten kann.

Diese vorgesehene dekomprimierte Form ist nicht zwingend die Form des Rotors im Betriebszustand, da dieser sich unter dem Einfluss des Fluiddrucks möglicherweise weiter verformt.

Eine besonders vorteilhafte Gestaltung des Rotors ist nun derart, dass der Rotor unter dem Einfluss des Fluiddrucks ausschließlich elastischen Verformungen unterliegt und im vorgesehenen Arbeitspunkt die für den Anwendungsfall optimale Geometrie aufweist.

Insgesamt erlaubt die erfindungsgemäße Gestaltung des Rotors einer Axialpumpe mit dem entsprechenden Schaufelblatt eine Material sparende und technisch einfache Herstellung des Rotors, die eine gute Komprimierbarkeit mit hoher Stabilität im Betrieb verbindet.

## Patentansprüche

1. Rotor für eine Axialpumpe zur Förderung eines Fluids mit einer Rotationsachse (10) und mit einem Schaufelblatt (12,12',122",12"'), das wenigstens eine Teilfläche aufweist, die sich quer zur Rotationsachse über diese hinweg erstreckt, wobei das Schaufelblatt Stege (15,15',15",15"',16,16',16",16"',17) aufweist, die jeweils einzeln oder als Netzwerk verschiedene Randbereiche des Schaufelblattes miteinander verbinden.

2. Rotor nach einem der Ansprüche 1 bis 10, **dadurch gekennzeichnet, dass** das Schaufelblatt (12,12',12'',12''') nabenfrei ist.

3. Rotor nach einem der Ansprüche 1 bis 11, **dadurch gekennzeichnet, dass** das Schaufelblatt (12,12',12",12"')selbsttragend ist.

4. Rotor nach Anspruch 1 oder einem der folgenden, **dadurch gekennzeichnet, dass** wenigstens ein Steg (15",15"',16,16",16"') oder das Netzwerk von Stegen zwei Randbereiche des Schaufelblattes (12,12",12"')über die Rotationsachse (10) hinweg verbindet.

5. Rotor nach Anspruch 1, **dadurch gekennzeichnet, dass** wenigstens ein Steg (15',16')oder das Netzwerk von Stegen zwei Randbereiche (20) des Schaufelblattes (12') verbindet, die in Längsrichtung der Rotationsachse gesehen einander gegenüberliegen.

6. Rotor nach Anspruch 1 oder einem der folgenden, **dadurch gekennzeichnet, dass** jeweils zwei oder mehr zueinander parallele oder in konstantem Abstand zueinander verlaufende Stege (15,15',15",15"',16,16', 16",16"',17) vorgesehen sind.

7. Rotor nach Anspruch 1 oder einem der folgenden, **dadurch gekennzeichnet, dass** wenigstens einer der Stege (15,16,17) mäanderförmig gestaltet ist.

8. Rotor nach Anspruch 1 oder einem der folgenden, **dadurch gekennzeichnet, dass** die Stege (15,15',15",15"',16,16',16",16"',17) aus einer Formgedächtnislegierung bestehen.

9. Rotor nach Anspruch 1 oder einem der folgenden, **dadurch gekennzeichnet, dass** Randbereiche (18,19, 20,20',20",21,21',21") des Schaufelblattes als Randleisten ausgebildet sind.

10. Rotor nach Anspruch 9, **dadurch gekennzeichnet, dass** das Schaufelblatt (12,12',12",12"') von einer durchgehenden Randleiste umgeben ist.

11. Rotor für eine Axialpumpe zur Förderung eines Fluids mit einer Rotationsachse (10) und mit einem Schaufelblatt (12,12',12",12"'), **dadurch gekennzeichnet, dass** das Schaufelblatt (12,12',12",12"') nabenlos als ein bezüglich seiner Kontur flacher Körper gestaltet ist, der um eine Achse spiralförmig verdreht ist.

12. Rotor nach Anspruch 11, **dadurch gekennzeichnet, dass** das Schaufelblatt als ein Gitterwerk oder Netzwerk von Stegen (15,15',15",15"',16, 16',16",16"',17) aus einem ebenen Blech hergestellt ist.

13. Rotor nach Anspruch 12, **dadurch gekennzeichnet, dass** das Schaufelblatt, insbesondere aus einem Nitinolblech, durch Ausschneiden der Stege (15,15',15",15"',16,16',16",16"',17), insbesondere durch Wasserschneiden, Laserschneiden oder Elektroerodieren hergestellt ist.

14. Rotor nach Anspruch 13, **dadurch gekennzeichnet, dass** die Stege (15,15',15",15"',16,16',16",16"',17) in der Blechebene und/oder senkrecht dazu mäanderförmig geformt sind.

15. Rotor nach Anspruch 13, **dadurch gekennzeichnet, dass** die Stege (15,15',15",15"',16,16',16",16"',17) in der Blechebene ein anderes Flächenträgheitsmoment aufweisen als senkrecht dazu.

16. Rotor nach Anspruch 1 oder einem der folgenden, **dadurch gekennzeichnet, dass** das Schaufelblatt (12,12',12",12"') mit einem dieses umgebenden hohlzylindrischen Bauteil (13) fest verbunden ist.

17. Rotor nach Anspruch 1 oder einem der folgenden, **dadurch gekennzeichnet, dass** zwischen den Stegen (15,15',15",15"',16,16',16",16"',17) und dem Rand des Schaufelblattes (12,12',12",12"') eine Folie gespannt ist.

18. Rotor nach Anspruch 1 oder einem der folgenden, **dadurch gekennzeichnet, dass** das Schaufelblatt (12,12',12",12"') gegebenenfalls mit dem hohlzylindrischen Bauteil (13) zusammen radial komprimierbar ist.
